# FASCICULE DE BREVET EUROPEEN

(11) **EP 0 818 529 B1**
(45) Date de publication et mention de la délivrance du brevet: **22.06.2005**
(21) Numéro de dépôt: 97201703.2
(22) Date de dépôt: 05.06.1997
(51) Int. Cl.: C12N 1/00

(54) **Procédé de séchage par pulvérisation**
Verfahren zur Sprühtrocknung
Spray-drying process

(30) Priorité: 09.07.1996 EP 96201922; 10.09.1996 EP 96202518
(43) Date de publication de la demande: 14.01.1998
(73) Titulaire: SOCIETE DES PRODUITS NESTLE S.A., 1800 Vevey (CH)
(72) Inventeur: Meister, Niklaus, 3506 Grosshoechstetten (CH); Aebischer, Jürg, 3097 Liebefeld (CH); Vikas, Martin, 3510 Konolfingen (CH); Eyer, Kurt, 3600 Thun (CH); De Pasquale, David, 3510 Konolfingen (CH)

(56) Documents cités:
- WO-A-91/01731
- DE-A- 2 626 626
- US-A- 4 702 799
- JOURNAL OF FOOD SCIENCE, vol. 62, no. 3, 1997, pages 576-585, XP002038914 B.C.S. TO AND M.R. ETZEL: "Spray drying, freeze drying or freezing of three different lactic acid bacteria species"
- JOURNAL OF FOOD SCIENCE, vol. 62, no. 1, 1997, pages 167-171, XP002038915 B.C.S. TO AND M.R. ETZEL: "Survival of B. linens (ATCC 9174) after spray drying, freeze drying or freezing"
- JOURNAL OF DAIRY SCIENCE, vol. 78, no. 4, 1995, pages 761-768, XP002038916 J.A.C. JOHNSON AND M.R. ETZEL: "Properties of L. helveticus CNRZ-32 attenuated by spray drying, freeze drying or freezing"
- JOURNAL OF APPLIED BACTERIOLOGY, vol. 78, 1995, pages 456-462, XP002038917 P. TEIXEIRA ET AL.: "Spray drying as a method for preparing concentrated cultures of L. bulgaricus"
- BIOTECHNOLOGY AND BIOENGINEERING, vol. XII, 1970, pages 135-140, XP002038918 T.P. LABUZA ET AL.: "Engineering factors in single cell protein production. II. Spray drying and cell viability"
- CANADIAN JOURNAL OF MICROBIOLOGY, vol. 23, no. 6, 1977, pages 716-720, XP002038919 A. CHOPIN ET AL.: "Destruction of M. lacticum, E. coli et S. aureus au cours du séchage du lait par atomisation. "
- BIOTECHNOLOGY AND BIOENGINEERING, vol. XVI, 1974, pages 1245-1259, XP002038920 H. ELIZONDO ET AL.: "Death kinetics of yeast in spray drying"

## Description

L'invention a pour objet un nouveau procédé de séchage par pulvérisation d'une composition comprenant des microorganismes.

### Etat de la technique

L'industrie a besoin, pour sécher des microorganismes, de disposer de procédés qui son faciles à mettre en oeuvre et qui sont économiques. Le séchage par pulvérisation consiste généralement à pulvériser dans une enceinte une suspension de microorganismes sous un flux d'air chaud, l'enceinte comprenant à cet effet une entrée d'air chauffé, une sortie pour l'évacuation de l'air et une sortie de récupération de la poudre de microorganismes séchés.

Le séchage de microorganismes par pulvérisation présente cependant l'inconvénient d'endommager, voire de tuer les microorganismes dès que la température de séchage est très élevée.

US3985901 (Instituto de Biologia Aplicada) explique en effet qu'une température de 180°C à 300°C, à l'entrée d'un dispositif de pulvérisation, est susceptible de tuer tous les organismes vivants. Ces observations sont également confirmées dans EP298605 (Unilever: page 2, lignes 43-48), et EP63438 (Scottish Milk Marke: page 1, lignes 14-21).

Certaines espèces de bactéries lactiques sont néanmoins naturellement thermorésistantes, c'est à dire capables de résister à des températures élevées. Chopin et al. ont ainsi montré que l'on peut sécher par pulvérisation à 215°C une culture sporulante de *Microbacterium lacticum* et obtenir un peu plus de 10% de survie après séchage (Canadian J. Microb., 23, 755-762, 1977). Malheureusement ces espèces font généralement parties de la flore contaminante des aliments qui est responsable de l'apparition de mauvais goûts. Ces bactéries lactiques thermorésistantes ne sont donc pas adaptées à l'alimentation humaine (*In* "Fundamentaly of Food Microbiology, Marion L. Fields, AVI Publishing Comp, Wsetport, 1979).

En conclusion, la température de séchage par pulvérisation est ainsi un des facteurs limitang la viabilité des microorganismes utilisés traditionnellement dans la fermentation de produits alimentaires. On peut d'ailleurs remarquer que tous les procédés classiques de séchage de microorganismes par pulvérisation, utilisent en pratique une température d'entrée d'air chauffé de l'ordre de 100-180°C. De plus, ces procédés recourent également à des agents de protection pour maintenir en vie les microorganismes séchés.

NL7413373 (DSO Pharmachim) décrit en effet un procédé de séchage par pulvérisation de céréales fermentés par des bactéries lactiques dans lequel, les températures d'entrée et de sortie d'air sont respectivement de 150°C et de 75°C.

J73008830 (Tokyo Yakult Seizo) décrit par ailleurs un procédé de séchage de microorganismes par pulvérisation, dans lequel on utilise une température d'entrée d'air de l'ordre de 120-155°C, une température de sortie d'air de l'ordre de 40-55°C, et des agents chimiques de protection.

J57047443 (Minami Nippon Rakun) décrit un procédé de séchage similaire où les températures d'entrée et de sortie d'air sont respectivement de l'ordre de 105-150°C et 55-70°C.

J02086766, J02086767, J02086768, J02086769 et J02086770 (tous de "Kubota") décrivent tous des procédés de séchage de microorganismes par pulvérisation dans lesquels, les températures d'entrée et de sortie d'air sont respectivement de l'ordre de 110-180°C et 70-75°C.

Enfin, SU724113 (Kiev Bacterial Prep.), SU1097253 (Protsishin et al.), SU1227145 (Protsishin et al.), SU1292706 (Appl. Biochem. Res.) et SU1581257 (Dairyland Food Labs.) décrivent tous aussi des procédés de séchage par pulvérisation d'une culture de bactéries dans lesquels, les températures d'entrée et de sortie d'air sont respectivement de l'ordre de 60-165°C et 30-75°C.

Il faut souligner quer le fait de limiter la température de séchage à moins de 200°C, lors d'un séchage de microorganismes par pulvérisation, limite d'autant le rendement du procédé. L'objectif de la présente invention est de pallier cet inconvénient.

Johnson J.A.C. et Etzel M.R. 1995, 78:761-768 décrivent un procédé de séchage dans lequel la température d'entrée d'air est de 220°C.

La publication de Labuza T.P. et al. 1970, 12 :135-140 concerne le séchage par pulvérisation de levures dans lequel la température d'entrée d'air est de 250°C.

### Résumé de l'invention

A cet effet, la présente invention concerne un procédé de séchage par pulvérisation dans lequel, on prépare une composition comprenant des microorganismes bénéfiques pour l'alimentation humaine, et on la réduit en poudre par pulvérisation dans un dispositif de séchage par pulvérisation ayant une température d'entrée d'air chauffé de 200-400°C et une température de sortie d'air de 40-90°C, le temps de résidence de la culture dans le dispositif étant ajusté de façon à obtenir au moins 1% de survie des microorganismes après séchage.

On a trouvé avec surprise qu'un dispositif de séchage par pulvérisation ayant une température d'entrée d'air supérieure à 200°C, et même supérieure à 300°C, n'endommage pas ou peu les microorganismes bénéfiques pour l'alimentation humaine, pour autant que le temps de résidence des gouttelettes dans le dispositif soit suffisamment court afin que la température interne des cellules ne devienne pas létale. Il a en effet été observé, que la température interne des gouttelettes pulvérisées peut ne pas excéder environ 40-70°C, à cause du refroidissement provoqué par l'évaporation de l'eau. L'invention réside ainsi dans la sélection des conditions opératoires pour que les gouttelettes pulvérisées arrivent sous une forme sèche uniquement à la sortie du dispositif de séchage.

On a constaté qu'un séchage très rapide des microorganismes favorise une bonne survie. L'utilisation de températures d'entrée d'air élevées, peut ainsi conduire à un séchage quasiment instantané.

On a également observé que l'on obtient d'excellentes survies des microorganismes lorsque l'on pulvérise conjointement une culture de microorganismes et une composition alimentaire.

### Description détaillée de l'invention

Pour mettre en oeuvre le présent procédé, on prépare une culture d'un microorganisme, qui peut être une bactérie, une levure, un champignon ou un mélange de ces microorganismes. L'homme du métier est en fait à même de sélectionner le milieu de culture qui est le plus adapté à la croissance de ces microorganismes.

De préférence, on prépare une culture d'au moins un microorganisme choisi dans le groupe formé par les bactéries lactiques bénéfiques pour la santé humaine notamment les bifidobactéries comme *Bifidobacterium infantis*, les lactocoques comme *Lactococcus lactis* subsp. *lactis, Lactococcus lactis* subsp. *cremoris*, *Lactococcus lactis* subsp. *lactic biovar diacetylactis*, les streptocoques comme *Streptococcus thermophilus, Streptococcus faecalis*, les lactobacilles comme *Lactobacillus delbrueckii* subsp. *bulgaricus*, *Lactobacillus acidophilus* (comprenant 6 sous-groupes dont *L. johnsonii*; voir Fujisawa et al., Int. J. Syst. Bact., 42, 487-491, 1992), *Lactobacillus helveticus*, *Lactobacillus farciminis*, *Lactobacillus alimentarius*, *Lactobacillus casei* subsp. *casei, Lactobacillus delbruckii* subsp. *lactis, Lactobacillus sake*, *Lactobacillus curvatus*, les pédiocoques comme *Pediococcus pentosaceus, Pediococcus acidilactici*, *Pediococcus halophilus*, les staphylocoques comme *Staphylococcus xylosus*, *Staphylococcus carnosus,* les microcoques comme *Micrococcus varians;* les levures notamment du genre *Debaromyces, Candida, Pichia, Torulopsis* et *Saccharomyces* comme *Debaromyces hansenii, Candida krusei, Pichia saitoi, Torulopsis holmii*, *Torulopsis versatilis*, *Torulopsis etchellsii*, *Saccharomyces cerevisiae* par exemple *S. cerevisiae* NCIMB 40612 décrit dans EP663441, *Saccharomyces rouxii;* et les champignions notamment du genre *Aspergillus*, *Rhizopus*, *Mucor* et *Penicillium* comme *Aspergillus oryzae*, *Aspergillus phoenicis, Aspergillus niger, Aspergillus awamori, Rhizopus oryzae, Rhizopus oligosporus, Rhizopus japonicus, Rhizopus formosaensis, Mucor circinelloides, Mucor japanicus, Penicillium glaucum* et *Penicillium fuscum.*

L'invention est particulièrement indiquée pour les microorganismes qui sont sensibles aux conditions de séchage par pulvérisation, notamment ceux qui sont sensibles à la chaleur (thermo-sensibles) et/ou à la présence d'air (anaéorobes préférentiels), par exemple. Parmi les microorganismes particulièrement sensibles, on peut compter les bactéries lactiques probiotiques. Dans le cadre de la présente invention, les bactéries probiotiques sont définies comme étant des bactéries lactiques qui sont capables d'adhérer aux cellules intestinales humaines, d'exclure des bactéries pathogènes sur des cellules intestinales humaines, et d'agir sur le système immunitaire humain en lui permettant de réagir plus fortement à des agréssions externes, par exemple en augmentant les capacités de phagocytose des granulocytes issus du sang humain (J. of Dairy Science, 78, 491-197, 1995).

A titre d'exemple, on peut utiliser la souche *Lactobacillus acidophilus* CNCM I-1225 décrite dans EP577904. Cette souche a été récemment reclassifié parmi les *Lactobacillus johnsonii*, suite à la nouvelle taxonomie, proposée par Fujisawa et *al*., qui fait maintenant autorité en matière de taxonomie des lactobacilles acidophiles (Int. J. Syst. Bact., 42, 487-791, 1992). D'autres bactéries probiotiques sont également disponibles, comme celles décrites dans EP199535 (Gorbach et al.), US5591428 (Bengmark et al.) ou dans US5296221 (Mitsuoka et al.), par exemple.

Cette culture de microorganismes peut comprendre, avant ou après fermentation, au moins un agent chimique de protection connu pour améliorer la survie des microorganisme pendant le séchage et/ou pendant la conservation de la poudre. L'homme du métier dispose d'une littérature abondante sur ces agents de protection. A cet effet, les agents de protection décrits dans les brevets US3897307, US4332790, J73008830, J57047443, J02086766, J02086767, J02086768, J02086769, J02086770, SU724113, SU1097253, SU1227145, SU1292706 et SU1581257 sont incorporés par référence dans la description de la présente invention. A titre d'indication, ces agents de protection peuvent être des vitamines comme l'acide ascorbique, des acides aminés ou leurs sels comme la lysine, la cystéine, la glycine et le glutamate de sodium, des protéines ou des hydrolysats de protéines pouvant provenir du lait ou du soja, des sucres comme le lactose, le tréhalose, le saccharose, la dextrine et la maltodextrine, et des graisses notamment une graisse de beurre (huile de beurre), de palme, d'arachide, de cacao, de colza ou de soja, par exemple. Enfin, ces agents de protection peuvent être ajoutés à la culture à raison de 0,1 à 80% en poids, par exemple.

La culture de microorganismes contient de préférence au moins 10⁷ colonies de cellules vivantes par gramme ou cfu/g (cfu est l'abréviation anglaise de "colony forming unit"). On peut choisir aussi de concentrer cette culture, par exemple par centrifugation, pour en augmenter le titre de cellules vivantes jusqu'à au moins 10⁸ cfu/g, de préférence 10⁸ - 10¹¹ cfu/g.

Si l'on veut une poudre constituée principalement de microorganismes, on peut directement sécher par pulvérisation la culture de microorganismes. Par contre, si l'on désire une composition alimentaire déshydratée, facilement dispersible dans de l'eau et comprenant des microorganismes vivants, il est préférable de sécher en même temps tous les composants de cette composition, plutôt que de la préparer en mélanger les différents constituants déjà sous formes secs. On évite ainsi la formation de grumeaux ou de précipités indésirables.

Dans une première mise en oeuvre pour préparer une composition alimentaire déshydratée, on peut ainsi mélanger la culture de microorganismes avec une composition alimentaire liquide, le cas échéant on peut concentrer le mélange jusqu'à une teneur en eau de l'ordre de moins de 70%, puis on peut sécher le mélange par pulvérisation dans les conditions de séchage selon l'invention. Cette mise en oeuvre est particulièrement indiquée pour les compositions déshydratées à base de lait comprenant des bactéries lactiques qui sont peu sensibles au séchage par pulvérisation, c'est à dire qui sont capables de survivre à raison d'au moins 10-50% dans les conditions de séchage selon l'invention. Plus particulièrement, on peut ainsi mélanger une culture de microorganismes à une composition alimentaire de sorte à obtenir un mélange dont au moins 80% en poids sec des constituents provient de la composition alimentaire, puis on peut sécher ledit mélange par pulvérisation dans les conditions de séchage selon l'invention.

Dans une deuxième mise en oeuvre pour préparer une composition alimentaire déshydratée, on peut aussi réduire en poudre dans le dispositif de séchage par pulvérisation, conjointement, une composition comprenant les microorganismes et une autre composition alimentaire. Cette mise en oeuvre est particulièrement indiquée pour les compositions déshydratée à base de lait comprenant des bactéries lactiques qui sont sensibles au séchage par pulvérisation, c'est à dire qui sont incapables de survivre à raison d'au moins 10-50% dans les conditions de séchage selon l'invention. Plus particulièrement, on peut sécher conjointement, c'est à dire en même temps et dans une même enceinte, 1 partie d'une culture de microorganismes et au moins 1 partie d'une composition alimentaire, notamment 1-1000 parties, lesdites parties étant calculées à l'état sec, par exemple.

De préférence, la composition alimentaire qui est utilisée pour préparer la composition alimentaire déshydratée, est une composition liquide dont au moins un des composants est choisi dans le groupe formé par le lait, la viande, le poisson, un fruit et un légume, par exemple. De préférence, on concentre la composition alimentaire, avant de la pulvériser, jusqu'à une teneur en eau de moins de 70 % en poids.

Cette composition alimentaire peut ainsi comprendre une partie finement divisée, cuite ou crue, provenant d'un végétal comestible, qu'il s'agisse d'une graine, racine, tubercule, tige, feuille, fleur ou fruit, par exemple. Parmi les végétaux préférés, on peut plus particulièrement distinguer les feuilles, notamment le poireau, l'asperge, le fenouil et le chou; les tiges, notamment la rhubarbe et le brocoli; les graines comme le cacao, le petit pois, le soja ou provenant des céréales; certaines racines, notamment la carotte, l'oignon, le radis, le céleri et la betterave; les tubercules, notamment le manioc et la pomme de terre; et les fruits notamment la tomate, la courgette, l'aubergine, la banane, la pomme, l'abricot, le melon, la pastèque, la poire, la prune, la pêche, la cerise, le kiwi, la baie d'argousier, les nèfles et la mirabelle, par exemple. On peut aussi utiliser comme végétaux des champignons supérieurs comestibles, notamment *Agaricus bisporus*, *Pleurotus ostreatus, Boletus edulis* ou *Lentinus edodes*, par exemple.

Cette composition alimentaire peut aussi comprendre une partie finement divisée, cuite ou crue, provenant d'un animal, qu'il s'agisse de lait, d'oeuf, de viande, de poisson, et/ou d'une de leur fraction, notamment une fraction protéique et/ou un hydrolysat de protéines, par exemple. Cette composition alimentaire peut être ainsi un lait de vache hydrolysé et hypoallergénique conforme à la directive Européenne 96/4/EC (Official Journal of the European Communities, No OJ L49/12, 1996), par exemple.

Les dispositifs de séchage par pulvérisation utilisés traditionnellement pour la fabrication industrielle d'une poudre de lait ou de café peuvent être particulièrement bien adaptés aux besoins de la présente invention (voir Jensen J.D., Food technology, June, 60-71, 1975). A titre d'exemple, on peut adapter facilement les dispositifs de séchage par pulvérisation décrit dans IE65390 (Charleville Res. LTD) et US4702799 (Nestlé).

De préférence, ces dispositifs présentent en fonctionnement une zone à très haute température (200-400°C) à l'extrémité de la buse de pulvérisation, ladite zone pouvant représenter jusqu'à 50% du volume de l'enceinte, de préférence 0,1% à 20%, le reste du dispositif présentant une température inférieure pouvant atteindre la température de sortie d'air, par exemple. Le dispositif décrit dans US3065076 (Nestlé) remplit particulièrement ces besoins.

De préférence, ces dispositifs présentent aussi en fonctionnement une entrée d'air secondaire, l'air secondaire ayant une température choisie pour ajuster la température de l'air à la sortie du dispositif. Cette entrée d'air secondaire peut être située à proximité de l'entrée d'air chauffé définie ci-dessus, par exemple.

Si l'on veut sécher conjointement une composition comprenant des microorganismes et une autre composition alimentaire, il faut prévoir au moins une buse de pulvérisation par composition. En fonctionnement, l'emplacement des buses de pulvérisation n'est pas critique. On peut ainsi pulvériser les deux compositions dans la zone à très haute température, par exemple. On peut aussi pulvériser la composition alimentaire dans la zone à très haute température, et en même temps pulvériser les microorganismes dans une zone ayant une température inférieure, par exemple.

L'invention réside en fait dans la sélection appropriée du temps de résidence des microorganismes dans le dispositif de séchage. De préférence, les gouttelettes pulvérisées arrivent sous une forme sèche vers la sortie du dispositif, c'est à dire là où la température de sortie est de 40-90°C, par exemple. Ce temps de résidence peut être ajusté à l'aide des différents paramètres réglant un dispositif de séchage par pulvérisation, comme la pression de pulvérisation des gouttelettes, la pression du flux d'air chaud, et/ou la distance que doivent parcourir les gouttelettes dans la chambre de séchage, par exemple. Il n'est pas possible de fournir des valeurs précises pour chaque paramètre impliqué dans l'ajustement du temps de résidence, puisque ces paramètres et leurs valeurs associés dépendent du type de dispositif de séchage par pulvérisation utilisé. A titre d'indication, la pression appliquée à l'extrémité des buses pulvérisant les microorganismes ou la composition alimentaire peut être comprise entre 5-250 bar, et la pression de l'air chaud à l'entrée du dispositif peut être comprise entre 100 et 200 mbar. Ainsi, pour simplifier la définition de cette ajustement du temps de résidence de la culture selon l'invention, on admettra que ce temps est conforme à la présente invention si le taux de survie des bactéries venant d'être séchés est d'au moins 1%, l'homme du métier étant en effet capable de sélectionner les paramètres opératoires appropriés pour atteindre ce résultat.

De préférence, le temps de résidence de la culture dans le dispositif de séchage est ajusté de façon à obtenir aussi une poudre ayant une Activité d'eau (Aw) à 25°C comprise entre 0,05 et 0,5. En effet, les meilleurs taux de survie après séchage et pendant la conservation sont obtenus pour une poudre ayant ce domaine d'activité d'eau.

De même les meilleurs taux de survie après séchage et pendant la conservation sont obtenus lorsque le dispositif de séchage présente au moins une des conditions suivantes, à savoir, une température d'entrée de 250-400°C, une température de sortie de 50-75°C, et un temps de résidence de la culture ajusté de façon à obtenir au moins 10% de survie après séchage.

D'autres paramètres peuvent aussi influencer la survie des microorganismes. Ainsi, l'humidité relative de l'air à la sortie du dispositif de séchage peut être de l'ordre de 10-40%, de préférence 20-40%. De plus, on peut introduire dans la composition ayant des microorganismes, avant la buse de pulvérisation, un gaz inerte utilisable dans les procédés alimentaires, notamment le CO₂, l'azote, l'argon, l'hélium, seuls ou en mélange, par exemple.

Si l'on sèche uniquement la culture de microorganismes, le présent procédé peut ainsi fournir une poudre de microorganismes ayant une densité de 200-1000 g/l mais de préférence 500-800 g/l, ayant une Aw à 25°C de 0,05- 0,5, ayant au moins 10⁷ cfu/g mais de préférence 10⁸ - 10¹¹ cfu/g, et présentant une survie des microorganismes d'au moins 10% par ans à 4-27°C, de préférence d'au moins 90% par an à 4-27°C, par exemple. Cette poudre de microorganismes peut être conservée à des températures de réfrigération ou de congélation, avant d'être utilisée comme *inoculum* pour la fermentation de produits alimentaires, cosmétiques ou pharmaceutiques. Cette poudre peut aussi être administrée directement par voie orale, ou mélangées à certains aliments solides ou liquides. Elle peut être mélangée au lait dont on remplit le biberon d'un nourrisson, ou même être mélangé à du lait en poudre, par exemple. Elle peut aussi être mélangée à d'autres aliments destinés à être administrée par voie entérale à un patient hospitalisé, par exemple.

De même, si l'on prépare une composition alimentaire déshydratée, le présent procédé peut ainsi fournir une poudre alimentaire, facilement dispersible, ayant une densité de l'ordre de 200-1000 g/l, ayant une Aw à 25°C de l'ordre 0,05-0,5, ayant 1 à 10⁹ cfu/g, et présentant une survie des microorganismes d'au moins 10% par an à 20°C.

La présente invention est décrite plus en détail ci-après à l'aide du complément de description qui va suivre, qui se réfère à des exemples de séchage de cultures de bactéries lactiques et de levures. Les pourcentages sont donnés en poids sauf indication contraire. Il va de soi, toutefois, que ces exemples sont donnés à titre d'illustration de l'objet de l'invention dont ils ne constituent en aucune manière une limitation.

### Exemples 1- 4

On sèche par pulvérisation une culture de la souche *Lactobacillus johnsonii* CNCM I-1225 d'origine humaine, décrite dans EP577904 (Société des Produits Nestlé) comme étant une souche probiotique survivant difficilement dans un milieu oxygéné.

Pour cela, on mélange 3% d'une pré-culture fraîche dans un milieu MRS de la souche CNCM I-1225 à du milieu MSK stérile comprenant du lait écrémé en poudre reconstitué à 10%, 0,1% d'extrait de levure commerciale, 0,5% de peptone et 0,1 % de Tween 80, puis on le fermente pendant 8 heures à 40°C, sans brassage.

On prépare ensuite une culture à grande échelle de cette souche, en fermentant un milieu MSK stérile comprenant du lait écrémé en poudre reconstitué à 10-25%, 0,1% d'extrait de levure commerciale, 0,5% de peptone et 0,1 % de Tween 80, avec 3% du mélange fermenté ci-dessus, à 40°C, jusqu'à un pH de 5,5 (environ 1-3 heures), avec un brassage de 30 rotations par min et sous une atmosphère de CO₂. On continue la fermentation à pH 5,5 par ajout d'une base alcaline pendant quelques heures. Puis on refroidit la culture à 15-20°C.

Dans les exemples 1 à 4, on ajoute à la culture 2% en poids d'acide ascorbique, et 1,25% en poids de glutamate de sodium. Puis on sèche par pulvérisation les différents mélanges, dans un dispositif adapté de celui décrit à la figure 1.c de US3065076, à la différence près que l'on n'utilise pas de dispositif d'agglomération; on recycle dans l'enceinte la poudre qui est allée dans le récupérateur de poussière associé au sécheur; on injecte de l'air secondaire ayant une température de 18-30°C (selon la température ambiante), à proximité de l'entrée d'air chauffé, au moyen d'une simple ouverture de l'enceinte sur le milieu extérieur; et on injecte du CO₂ et/ou de l'azote dans la culture juste avant de la pulvériser.

Il faut en outre noter que la poudre est récupérée sur un lit fluidisé passant par 3 compartiments, les deux premiers compartiments servant à sécher encore plus la poudre à des températures de 60-90°C, et le dernier compartiment servant à refroidir la poudre à environ 30°C. Les conditions opératoires sont décrites dans le tableau 1 ci-dessous.

Après séchage, on récupère la poudre, on en dilue une partie dans de l'eau stérile, et on l'on étale sur un milieu MRS-agar (De Man et al, 1960) pour y énumérer le nombre de bactéries survivantes.

On détermine également l'activité d'eau de la poudre, définie par le rapport entre la pression de vapeur partielle de l'eau à la surface de la poudre et la pression de vapeur de l'eau pure à la même température. On peut déterminer l'Aw par la mesure de l'humidité relative d'équilibre atteinte dans une enceinte fermée à température constante. Pour cela, un échantillon de quelques g de poudre est enfermé dans un récipient étanche placé dans une chambre thermostatée à 25°C. L'espace vide autour de cet échantillon atteint à l'équilibre, au bout de 30-60 min, la même valeur Aw que l'échantillon. Un capteur électronique, monté dans le couvercle de fermeture du récipient, mesure alors l'humidité de cet espace vide par l'intermédiaire d'une résistance électrolytique.

On emballe les différentes poudres de microorganismes dans des récipients scellés comprenant une atmosphère d'azote et/ou de CO₂, on conserve chaque récipient à 20°C ou 27°C, pendant 12 mois, on détermine périodiquement le nombre de bactéries survivantes, puis on calcule le nombre de mois (valeur D) nécessaires théoriquement pour perdre 90% des bactéries lactiques à 20°C ou 27°C.

Pour comparaison, dans des conditions de stockages identiques, on mesure la survie de lots de bactéries CNCM I-1225 lyophilisées traditionnellement (par Hansen, DK), et on calcule le nombre de mois (valeur D) nécessaires théoriquement pour perdre 90% des bactéries lactiques à 20°C ou 27°C.

**Tableau 1**

| Conditions opératoires | Exemple 1 | Exemple 2 | Exemple 3 | Exemple 4 |
|---|---|---|---|---|
| % matière sèche | 27,31 | 13,13 | 27,89 | 26,75 |
| pH | 6,12 | 5,8 | 5,83 | 6,80 |
| Gaz (l./min) | 5,6 (CO₂) | 2,2 (N₂) | 3,8 (CO₂) | 6,4 (N₂/CO₂) |
| Pression de pulvérisation (bar) | 65 | 230 | 78 | 201 |
| Air à l'entrée (°C) | 317 | 310 | 320 | 309 |
| Pression de l'air chaud (mbar) | 160 | 130 | 160 | 130 |
| Air à la sortie (°C) | 64 | 60 | 71 | 72 |
| Humidité de l'air à la sortie (%) | 21 | 20 | 21 | 28 |
| Humidité de la poudre (%) | 2,88 | 3,19 | 3,90 | 3,71 |
| Activité d'eau de la poudre (Aw) | 0,182 | 0,071 | 0,147 | 0,143 |
| Rendement en poudre (kg/h) | 67 | 37 | 72 | 123 |
| Densité de la poudre (g/l) | 520 | 400 | 500 | 310 |
| Cfu/ml avant pulvérisation | 5,2 x 10⁸ | 8 x 10⁸ | 5,8 x 10⁸ | 5,9 x 10⁸ |
| Cfu/g après pulvérisation | 2,2 x 10⁸ | 9,65 x 10⁸ | 2,7 x 10⁸ | 3,2 x 10⁸ |
| Perte de viabilité (log cfu/g) | 0,92 | 0,79 | 0,87 | 0,82 |
| Viabilité après séchage (%) | 12,02 | 16,21 | 13,48 | 15,14 |
| Valeur D (mois) à 20°C | >12 | >12 | >12 | >12 |
| Valeur D (mois) à 27°C | >12 | >12 | >12 | >12 |
| Valeur D pour un lot de bactérie CNCM I-1225 lyophilisée conservé à 20°C: 10.1 mois | | | | |
| Valeur D pour un lot de bactérie CNCM I-1225 lyophilisée conservé à 27°C: 6.6 mois | | | | |

Les résultats, présentés dans le tableau 1 ci-dessus, montrent que l'on peut obtenir plus de 16% de survie des bactéries lactiques directement après séchage, et une stabilité remarquable des bactéries lactiques après stockage à des températures élevées.

### Exemple 5

On sèche par pulvérisation une culture de la souche *Saccharomyces cerevisae* NCIMB 40612 décrite dans EP663441 (Nestlé).

Pour cela, on réalise une fermentation de la souche NCIMB 40612 selon le processus fed-batch traditionnel en incubant à 30°C sous agitation (250 à 450 rpm) et aération croissantes (0,02 à 0,8 m³/h), pendant 24 heures, en maintenant le pH à 4,5 par ajout de quantités adéquates de NH₄OH, en contrôlant la mousse produite par ajout de quantités croissantes d'agent anti-moussant Contraspum 210 (1,5% poids/volume de milieu; Binggeli-Chemie, Suisse), et en ajoutant régulièrement une quantité croissante adéquate de milieu "mélasse" (84,85% de mélasse stérile, 13,85% d'eau, 1% H2SO4).

Puis on sèche les levures dans les mêmes conditions que celles décrites à l'exemple 2.

### Exemple 6

Cet exemple vise à montrer que la pulvérisation d'une composition alimentaire comprenant moins de 25% en poids d'une culture de bactéries lactiques probiotiques, peut donner de moins bonnes survies que celles obtenues aux exemples 7 à 9 lorsque l'on co-pulvérise une culture de bactéries probiotiques et une composition alimentaire.

On prépare un lait fermenté comme décrit aux exemples 1 à 4, on y ajoute 2% en poids d'acide ascorbique, 1,25% en poids de glutamate de sodium, et 300% en poids de lait concentré ayant 50% en poids de matière sèche, puis on sèche par pulvérisation le mélange, avec le dispositif décrit aux exemples 1-4, et avec les conditions opératoires décrites dans le tableau 2 ci-dessous. Comme décrit aux exemples 1 à 4, après séchage, on énumère le nombre de bactéries survivantes. Les résultats sont présentés dans le tableau 2 ci-après.

### Exemples 7-9

On sèche par pulvérisation, conjointement, du lait et une culture de la souche *Lactobacillus johnsonii* CNCM I-1225.

Pour cela, on prépare une culture de bactérie comme décrit aux exemples 1 à 4, on y ajoute des agents de protection, et on co-pulvérise en continu 1 partie de cette culture de bactéries avec environ 40 à 100 parties de lait concentré ayant 50% en matière sèche, lesdites pulvérisation étant réalisées conjointement dans des dispositifs adaptés de celui décrit à la figure 1.c de US3065076.

Comme décrit aux exemples 1 à 4, après pulvérisation, on récupère la poudre sur un lit fluidisé passant par 3 compartiments, les deux premiers compartiments servant à sécher encore plus la poudre, à des températures de 60-90°C, et le dernier compartiment servant à refroidir la poudre à environ 30°C. On énumère ensuite le nombre de bactéries survivantes dans la poudre alimentaire déshydratée, en tenant compte de la dilution réalisée avec le lait.

Les résultats sont présentés dans le tableau 2 ci-après. Les différentes poudres présentent en outre des stabilités dans le temps qui sont similaires à celles obtenues pour les poudres de microorganismes décrits aux exemples 1 à 4.

A l'exemple 7, on effectue conjointement les deux pulvérisations dans le dispositif représenté à la figure 1.c de US3065076, à la différence près que l'on n'utilise pas de dispositif d'agglomération. On recycle dans l'enceinte la poudre qui est allée dans le récupérateur de poussière. On injecte de l'air secondaire ayant une température de 18-30°C (selon la température ambiante), à proximité de l'entrée d'air chauffé, au moyen d'une simple ouverture de l'enceinte sur le milieu extérieur. On injecte du CO₂ dans la culture juste avant de la pulvériser. Et on pulvérise conjointement la culture et le lait à l'aide de deux buses dont les extrémités sont placées, dans l'enceinte, au niveau de l'entrée d'air chauffé (même emplacement que la buse 14 de la figure 1.c de US3065076). Les conditions opératoires sont décrites dans le tableau 2 ci-dessous.

Aux exemples 8-9, on effectue conjointement les deux pulvérisations dans le dispositif représenté à la figure 1.c de US3065076, à la différence près que l'on n'utilise pas de dispositif d'agglomération; on recycle dans l'enceinte la poudre qui est allée dans le récupérateur de poussière, l'entrée de la poudre recyclée se faisant à mi-hauteur de l'enceinte; on injecte de l'air secondaire ayant une température de 18-30°C (selon la température ambiante), à proximité de l'entrée d'air chauffé, au moyen d'une simple ouverture de l'enceinte sur le milieu extérieur; et on pulvérise le lait à l'aide d'une buse dont l'extrémité est placée, dans l'enceinte, au niveau de l'axe et de l'extrémité de l'entrée d'air chauffé (même emplacement que la buse 14 de la figure 1.c de US3065076). Simultanément, on pulvérise la culture de bactérie à l'aide d'une buse dont l'extrémité est placée, dans l'enceinte, au niveau de l'axe et de l'extrémité de l'entrée de la poudre recyclée. Les conditions opératoires sont décrites dans le tableau 2 ci-dessous.

**Tableau 2**

| Conditions opératoires | Exemple 6 | Exemple 7 | Exemple 8 | Exemple 9 |
|---|---|---|---|---|
| Culture de bactéries | | | | |
| Agents de protection | *Lait+A+GS | * L+A +T | * L+A+SG | * L+A+T |
| % matière sèche | 41,82 | 31,08 | 28,79 | 31,08 |
| pH | 6,3 | 6,15 | 6,48 | 6,15 |
| Gaz (l./min) | 6,5 (CO₂) | 2,5 (CO₂) | --- | --- |
| Débit (l./h.) | 496,3 | 78 | 30 | 53 |
| Pression de pulvérisation (bar) | 59 | 70 | 8 (buses à deux phases: N₂) | 8 (buses à deux phases: N₂) |

| Lait | | | | |
|---|---|---|---|---|
| % matière sèche | -- | 46,88 | 46,88 | 46,88 |
| Débit (kg/h.) | -- | 378 | 556 | 420 |
| Pression de pulvérisation (bar) | -- | 30 | 48 | 38 |
| Air à l'entrée (°C) | 310 | 309 | 310 | 305 |
| Pression de l'air chaud (mbar) | 190 | 164 | 190 | 160 |
| Air à la sortie (°C) | 65 | 65 | 64 | 65 |
| Humidité de l'air à la sortie (%) | 20,7 | 20 | 24,2 | 20,6 |
| Humidité de la poudre (%) | 3,3 | 3,5 | 3,8 | 4,0 |
| Rendement en poudre (kg/h) | 215 | 209 | 280 | 220 |
| Densité de la poudre (g/l) | 440 | 535 | 335 | 320 |
| Cfu/ml avant pulvérisation | 1,2 x 10¹⁰ | 4,45 x 10⁹ | 9,63 x 10⁹ | 5,81 x 10⁹ |
| Cfu/g après pulvérisation | 5,3 x 10⁶ | 6 x 10⁷ | 6,5 x 10⁷ | 8,2 x 10⁷ |
| Perte de viabilité (log cfu/g) | 3,72 | 1,42 | 1,19 | 1,23 |
| Viabilité après séchage (%) | <0,1 | 3,8 | 6,45 | 5,88 |
| *Lait +A + GS: 300% de lait concentré ayant 50 % en matière sèche + 2 % d'acide ascorbique + 1,25% de glutamate de sodium | | | | |
| *L + A + GS: 100% de lait concentré ayant 50 % en matière sèche + 2 % d'acide ascorbique + 1,25% de glutamate de sodium | | | | |
| *L + A + T: 100% de lait concentré ayant 50 % en matière sèche + 5 % d'acide ascorbique + 5% de tréhalose | | | | |

### Exemple 10

On pulvérise conjointement, dans les conditions décrites à l'exemple 8, une culture de bactéries lactiques CNCM I-1225 comprenant 5% d'acide ascorbique et 5% de tréhalose, et un jus de tomate concentré finement divisé ayant 50% de matière sèche.

### Exemple 11

On pulvérise conjointement, dans les conditions décrites à l'exemple 8, une culture de bactéries lactiques CNCM I-1225 comprenant 5% d'acide ascorbique et 5% de tréhalose, et un lait végétal à base de soja ayant 50% de matière sèche.

## Revendications

1. Procédé de séchage par pulvérisation, dans lequel on prépare une composition comprenant des microorganismes bénéfiques pour l'alimentation humaine, et on la réduit en poudre par pulvérisation dans un dispositif de séchage par pulvérisation ayant une température d'entrée d'air chauffé de 250-400°C et une température de sortie d'air de 50-75°C, le temps de résidence de la composition dans le dispositif étant ajusté de façon à obtenir au moins 10% de survie des microorganismes après séchage.

2. Procédé selon la revendication 1, dans lequel on concentre la composition avant de la réduire en poudre.

3. Procédé selon la revendication 2, dans lequel après concentration, la composition comprend moins de 70% d'eau.

4. Procédé selon la revendication 1, dans lequel le temps de résidence de la composition dans le dispositif de séchage est ajusté de façon à obtenir une poudre ayant une Aw à 25°C comprise entre 0,05 et 0,5.

5. Procédé selon la revendication 1, dans lequel la composition comprend au moins un agent de protection choisi dans le groupe formé par les vitamines comme l'acide ascorbique, les acides aminés ou leurs sels comme la lysine, la cystéine, la glycine et le glutamate de sodium, les protéines ou des hydrolysats de protéines pouvant provenir du lait ou du soja, les sucres comme le lactose, le tréhalose, le saccharose, la dextrine et la maltodextrine, et les graisses notamment les graisses de beurre, de palme, d'arachide, de cacao, de colza ou de soja.

6. Procédé selon la revendication 1, dans lequel la composition comprend au moins 80% en poids sec d'une composition alimentaire.

7. Procédé selon la revendication 1, **caractérisé en ce que** dans le dispositif de séchage par pulvérisation, on pulvérise conjointement la composition comprenant des microorganismes et une autre composition alimentaire.

8. Procédé selon la revendication 7, dans lequel on pulvérise conjointement 1 partie d'une culture de microorganismes et au moins 1 partie d'une composition alimentaire, lesdites parties étant calculées à l'état sec.

## Patentansprüche

1. Sprühtrocknungsverfahren, bei dem man eine Zusammensetzung herstellt, die für die menschliche Ernährung günstige Mikroorganismen enthält, und sie in einer Sprühtrocknungsvorrichtung mit einer Heißlufteintrittstemperatur von 250-400°C und einer Luftaustrittstemperatur von 50-75°C durch Zerstäubung zu Pulver zerkleinert, wobei die Verweilzeit der Zusammensetzung in der Vorrichtung so eingestellt wird, dass mindestens 10 % der Mikroorganismen nach Trocknung überleben.

2. Verfahren nach Anspruch 1, bei dem man die Zusammensetzung vor der Zerkleinerung zu Pulver konzentriert.

3. Verfahren nach Anspruch 2, bei dem die Zusammensetzung nach der Konzentration weniger als 70 % Wasser enthält.

4. Verfahren nach Anspruch 1, bei dem die Verweilzeit der Zusammensetzung in der Trocknungsvorrichtung so eingestellt wird, dass man ein Pulver mit einem Aw-Wert bei 25°C von 0,05 bis 0,5 erhält.

5. Verfahren nach Anspruch 1, bei dem die Zusammensetzung mindestens ein Schutzmittel enthält, das aus der Gruppe ausgewählt ist, die aus Vitaminen wie Ascorbinsäure, Aminosäuren oder ihren Salzen wie Lysin, Cystein, Glycin und Natriumglutamat, wobei die Proteine oder Proteinhydrolysate von Milch oder Soja stammen können, Zucker wie Lactose, Trehalose, Saccharose, Dextrin und Maltodextrin und Fette, insbesondere Butterfett, Palmfett, Erdnussfett, Kakaofett, Kolzafett oder Sojafett, besteht.

6. Verfahren nach Anspruch 1, bei dem die Zusammensetzung mindestens 80 Trockengew.-% einer Nahrungsmittelzusammensetzung enthält.

7. Verfahren nach Anspruch 1, **dadurch gekennzeichnet, daß** man in der Sprühtrocknungsvorrichtung gleichzeitig die Mikroorganismen enthaltende Zusammensetzung und eine andere Nahrungsmittelzusammensetzung zerstäubt.

8. Verfahren nach Anspruch 7, bei dem man gleichzeitig 1 Teil einer Kultur von Mikroorganismen und mindestens einen Teil einer Nahrungsmittelzusammensetzung zerstäubt, wobei diese Teile im trockenen Zustand gerechnet werden.

## Claims

1. A spray drying process in which a composition comprising microorganisms beneficial to human nutrition is prepared and is converted into a powder by spraying in a spray drying apparatus having a heated air inlet temperature of 250-400°C and an air output temperature of 50-75°C, the residence time of the composition in the apparatus being adjusted such that at least 10% survival of the microorganisms is obtained after drying.

2. A process according to claim 1, in which the composition is concentrated before being converted into a powder.

3. A process according to claim 2, in which, after concentration, the composition comprises less than 70% water.

4. A process according to claim 1, in which the residence time of the composition in the drying apparatus is adjusted such that a powder having an Aw at 25°C of between 0.05 and 0.5 is obtained.

5. A process according to claim 1, in which the composition comprises at least one protection agent selected from the group comprising vitamins such as ascorbic acid, amino acids or the salts thereof such as lysine, cysteine, glycine and sodium glutamate, proteins or protein hydrolysates which may originate from milk or soya, sugars such as lactose, trehalose, sucrose, dextrin and maltodextrin, and fats, in particular butter, palm, peanut, cocoa, rapeseed or soya fats.

6. A process according to claim 1, in which the composition comprises at least 80% by dry weight of a food composition.

7. A process according to claim 1, **characterised in that** the composition comprising microorganisms and another food composition are sprayed jointly in the spray drying apparatus.

8. A process according to claim 7, in which 1 part of a microorganism culture and at least 1 part of a food composition are sprayed jointly, said parts being calculated in the dry state.
